## Europäisches Patentamt
## European Patent Office
## Office européen des brevets

(11) Veröffentlichungsnummer: **0 168 109**
**B1**

(12)

# EUROPÄISCHE PATENTSCHRIFT

(45) Veröffentlichungstag der Patentschrift:
**21.09.88**

(51) Int. Cl.⁴: **A 61 B 6/00,** A 61 B 6/10

(21) Anmeldenummer: **85201076.8**

(22) Anmeldetag: **04.07.85**

(54) **Röntgenuntersuchungsgerät mit einem C- oder U-förmigen Träger für den Röntgenstrahler und die Bildaufnahmevorrichtung.**

(30) Priorität: **12.07.84 DE 3425650**

(43) Veröffentlichungstag der Anmeldung:
**15.01.86 Patentblatt 86/3**

(45) Bekanntmachung des Hinweises auf die Patenterteilung:
**21.09.88 Patentblatt 88/38**

(84) Benannte Vertragsstaaten:
**DE FR GB**

(56) Entgegenhaltungen:
**DE-A-1 466 880**
**DE-B-1 064 679**

(73) Patentinhaber: **Philips Patentverwaltung GmbH, Wendenstrasse 35 Postfach 10 51 49, D-2000 Hamburg 1 (DE)**

(84) Benannte Vertragsstaaten: **DE**

(73) Patentinhaber: **N.V. Philips' Gloeilampenfabrieken, Groenewoudseweg 1, NL- 5621 BA Eindhoven (NL)**

(84) Benannte Vertragsstaaten: **FR GB**

(72) Erfinder: **Christiansen, Dieter, Dorfstrasse 44, D-2071 Schönberg (DE)**

(74) Vertreter: **Hartmann, Heinrich, Dipl.- Ing., Philips Patentverwaltung GmbH Wendenstrasse 35 Postfach 10 51 49, D-2000 Hamburg 1 (DE)**

LIBER, STOCKHOLM 1988

## Beschreibung

Die Erfindung betrifft ein Röntgenuntersuchungsgerät mit einem C- oder U-förmigen Träger, an dessen Enden - aufeinander zentriert - ein Röntgenstrahler und eine Bildaufnahmevorrichtung angebracht sind und der um eine zur Verbindungslinie zwischen Strahler und Bildaufnehmer senkrechte Achse schwenkbar ist.

Solche Geräte sind seit langem bekannt. Dabei umgreifen die beiden Enden des Trägers mit dem daran befestigten Röntgenstrahler und der Bildaufnahmevorrichtung, meist einem Bildverstärker, den Tisch mit dem Patienten von einer der Längsseiten her wie eine Klammer, so daß eine Rotationsbewegung des Trägers um eine horizontale Achse - bei der Röntgenstrahler und Bildaufnahmevorrichtung stets aufeinander zentriert bleiben - nur bis zur Kollision mit dem Tisch oder dem Patienten möglich ist. Aus diesem Grund ist ein Wechsel von der Untertischtechnik (bei der der Röntgenstrahler sich unter dem Tisch befindet) zur Obertischtechnik (bei der sich die Röntgenröhre über dem Tisch befindet) bei derartigen Geräten allenfalls dann möglich, wenn entweder das Stativ, an dem der C- oder U-förmige Träger befestigt ist, oder der Tisch mit dem Patienten genügend weit seitlich verfahrbar sind.

Aufgabe der vorliegenden Erfindung ist es, ein Röntgenuntersuchungsgerät der eingangs genannten Art so auszugestalten, daß ein Wechsel von der Untertischtechnik zur Obertischtechnik ohne seitliche Verschiebung des Tisches oder des Statives möglich ist, an dem der C- oder U-förmige Träger befestigt ist.

Diese Aufgabe wird erfindungsgemäß dadurch gelöst, daß der Träger zwei Arme umfaßt, von denen der eine den Röntgenstrahler und der andere die Bildaufnahmevorrichtung trägt, daß die beiden Arme um zur Verbindungslinie wenigstens annähernd parallele Achsen schwenkbar gelagert und so miteinander gekoppelt sind, daß bei einer Drehung des einen Armes der andere mit der gleichen Winkelgeschwindigkeit aber mit entgegengesetztem Drehsinn gedreht wird. Anstelle eines starren Trägers benutzt die Erfindung also einen Träger mit zwei relativ zueinander drehbaren Armen. Wenn die beiden Arme dieses Trägers aus der Aufnahmestellung, bei der Röntgenstrahler und Bildaufnahmevorrichtung aufeinander zentriert sind, um 90° um die beiden Drehachsen gedreht werden, kann der Träger insgesamt um 180° gedreht werden, so daß ein Wechsel von der Untertischtechnik zur Obertischtechnik (und umgekehrt) möglich ist, ohne seitliche Verschiebung der Tischplatte bzw. das den Träger tragenden Statives.

Eine bevorzugte Weiterbildung der Erfindung sieht vor, daß der Abstand der Drehachsen der Arme von der Verbindungslinie so bemessen ist, daß bei horizontaler Lage der Drehachsen die Drehmomente der Arme sich gerade kompensieren. Die beiden Arme befinden sich dabei in jeder Stellung im Gleichgewicht, so daß sie ohne motorische Unterstützung durch den Benutzer gedreht werden können. Eine bevorzugte Weiterbildung der Erfindung sieht vor, daß die Arme über zwei Zahnräder mit dem gleichen Durchmesser miteinander gekoppelt sind. Das Gleichgewicht zwischen den beiden Armen läßt sich dabei durch geeignete Wahl der Zahnraddurchmesser erreichen.

Nach einer anderen Weiterbildung der Erfindung ist der Röntgenstrahler um eine zur Drehachse eines der Arme senkrechte Achse drehbar mit diesem Arm verbunden. Diese Weiterbildung gestattet es, unter Verwendung des Röntgenstrahlers und einer anderen Bildaufnahmevorrichtung, z. B. einer Kassette, Aufnahmen mit seitlichen Strahlengang anzufertigen.

Die Erfindung wird nachstehend anhand der Zeichnung näher erläutert. Es zeigen

Fig. 1 ein erfindungsgemäßes Röntgenuntersuchungsgerät und die
Fig. 2 bis 5 dieses Gerät bei unterschiedlicher Lage der Arme.

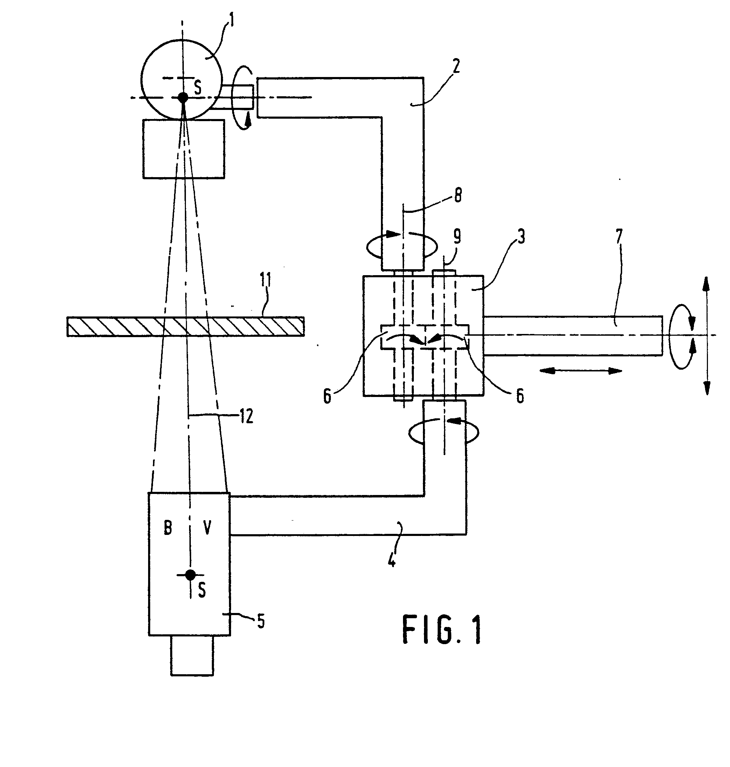

Ein Röntgenstrahler 1 ist um eine horizontale Achse drehbar an einem rechtwinkligen Arm 2 befestigt. Der Arm 2 ist in einem Gehäuse 3 gelagert, das auch einen rechtwinkligen Arm 4 trägt, an dem ein Bildverstärker 5 befestigt ist, der leichter ist als der Röntgenstrahler. In der in Fig. 1 dargestellten Stellung bilden die beiden Arme einen C- oder U-förmigen Bogen durch den Röntgenstrahler 1 und Bildverstärker aufeinander zentriert werden. In dieser Stellung verläuft die Verbindungslinie 12 zwischen Röntgenstrahler und Bildverstärker vertikal.

Der Arm 2 ist in dem Gehäuse 3 um eine vertikale Achse 8 drehbar gelagert, während der Arm 4 um eine vertikale Achse 9 drehbar ist. Die Achsen 8 und 9 befinden sich mit der Verbindungslinie 12 in einer Ebene. Die beiden Arme sind fest mit je einem von zwei im Gehäuse 3 angeordneten und miteinander in Eingriff stehenden Zahnrädern 6 verbunden, die den gleichen Durchmesser aufweisen. Wie durch Pfeile angedeutet, wird daher bei einer Drehung des Armes 2 und des mit ihm verbundenen Zahnrades 6 im Gegenuhrzeigersinn das mit dem Arm 4 verbundene Zahnrad und damit dieser Arm selbst im Uhrzeigersinn gedreht, wobei die beiden Drehwinkel dem Betrage nach jedoch gleich sind.

Die Abstände der beiden Drehachsen 8 und 9 voneinander, d.h. die Durchmesser der Zahnräder 6, sind so bemessen, daß die dem Produkt aus dem Abstand des Schwerpunktes S von der zugehörigen Achse und der im Schwerpunkt S wirksamen Last entsprechenden Momente einander gleich sind. Da die beiden Zahnräder immer im Eingriff sind und die auf sie wirkenden Momente immer gleich jedoch gegenläufig gerichtet sind, befinden sich die beiden Arme in jeder Stellung des Trägers im Gleichgewicht. Das Gehäuse 3, in dem die den C- bzw. U-förmigen Träger bildenden Arme 2 und 4 gelagert sind, wird von einem horizontalen, um seine Mittelachse 7 drehbaren Tragarm getragen, der derart mit dem Gehäuse verbunden ist, daß die von Röntgenstrahler und Bildverstärker auf die Mittelachse ausgeübten Momente sich ebenfalls gerade aufheben. Auf diese Weise wird erreicht, daß in jeder beliebigen Winkelstellung Gleichgewicht herrscht und die Arme 2, 4 um die Achsen 8, 9 bzw. gemeinsam um die Achse 7 ohne besonderen Kraftaufwand von Hand geschwenkt werden können.

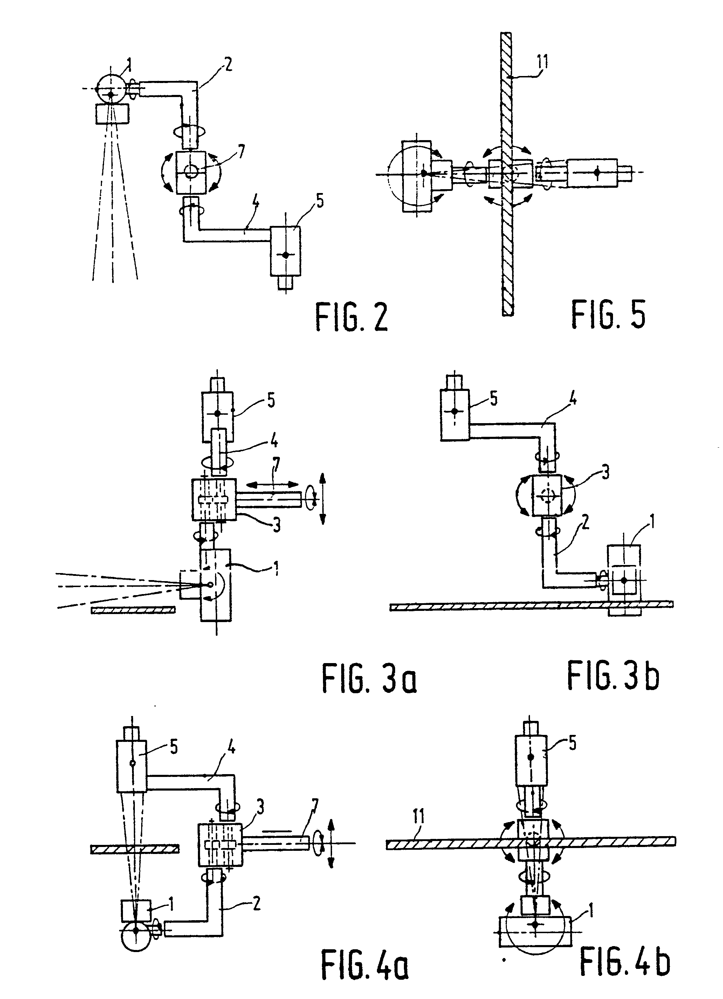

Der Tragarm 7 ist in vertikaler Richtung verfahrbar an einem nicht näher dargestellten Stativ befestigt, das seinerseits in Längsrichtung der horizontalen Tischplatte 11, d.h. senkrecht zur Zeichenebene, und quer dazu so weit verfahren werden kann, daß ein auf dem Tisch 11 befindlicher Patient von Kopf bis Fuß sowie auf beiden Seiten durchstrahlt werden kann. Wenn die Arme 2 und 4 um je 90° aus der in Fig. 1 dargestellten Position um die Achsen 8 bzw. 9 gedreht werden, geht der Träger, wie in Fig. 2 dargestellt, aus einer C- bzw. U-Form in eine S-Form über. Röntgenstrahler 1 und Bildverstärker 5 befinden sich dann seitlich von der Tischplatte, und es können dann Röntgenaufnahmen neben der Tischplatte mit dem Röntgenstrahler 1 und einem anderen Bildaufnehmer, z. B. einer Kassette, angefertigt werden. Außerdem kann die Einheit dann um die Achse 7 gedreht werden, so daß die in den Fig. 3a und 3b dargestellte Position einnimmt, wenn der Tragarm 7 angehoben und der Röntgenstrahler 1 bezogen auf den Tragarm 2 um 90° gedreht worden ist, so daß mit ihm und einem seitlich der Tischplatte angebrachten Bildaufnehmer eine Röntgenaufnahme mit seitlichem Strahlengang angefertigt werden kann.

Wenn dann der Tragarm 7 wieder abgesenkt, der Röntgenstrahler 1 wieder zurückgedreht und die beiden Arme 2 und 4 um die Achsen 8 und 9 in ihre ursprüngliche Stellung zurückgedreht werden, ergibt sich die in den Fig. 4a und 4b dargestellte Stellung. Diese unterscheidet sich von der Stellung nach Fig. 1 dadurch, daß der Röntgenstrahler sich nicht mehr oberhalb, sondern unterhalb der Tischplatte befindet und umgekehrt der Bildverstärker oberhalb statt unterhalb. Ebenso ist es möglich, die Tischplatte senkrecht zu stellen und den Träger 2, 4 um die Mittelachse des Tragarmes 7 um 90°- bezogen auf die Position nach Fig. 1 oder Fig. 4 - zu drehen, wobei sich die Stellung nach Fig. 5 ergibt, in der der Strahlengang horizontal verläuft. Da sich die vom Röntgenstrahler und vom Bildverstärker bezüglich der dann horizontal verlaufenden Achsen 8 und 9 ausgeübten Momente aus den eingangs erwähnten Gründen gerade kompensieren, ist auch in dieser Stellung eine leichte Handhabung des Gerätes möglich.

Die Geräteteile sind in beliebigen Positionen auf nicht näher dargestellte, an sich bekannte Weise arretierbar.

## Patentansprüche

1. Röntgenuntersuchungsgerät mit einem C- oder U-förmigen Träger, an dessen Enden - aufeinander zentriert - ein Röntgenstrahler (1) und eine Bildaufnahmevorrichtung (5) angebracht sind und der um eine zur Verbindungslinie (12) zwischen Strahler und Bildaufnehmer senkrechte Achse (7) schwenkbar ist, dadurch gekennzeichnet, daß der Träger zwei Arme (2, 4) umfaßt, von denen der eine den Röntgenstrahler (1) und der andere die Bildaufnahmevorrichtung (5) trägt, daß die beiden Arme um zur Verbindungslinie (12) wenigstens annähernd parallele Achsen (8, 9) schwenkbar gelagert und so miteinander gekoppelt sind, daß bei einer Drehung des einen Armes der andere mit der gleichen Winkelgeschwindigkeit aber mit entgegengesetztem Drehsinn gedreht wird.

2. Röntgenuntersuchungsgerät nach Anspruch 1, dadurch gekennzeichnet, daß der Abstand der Drehachsen (8, 9) der Arme von der Verbindungslinie (12) so bemessen ist, daß bei horizontaler Lage der Drehachsen (8, 9) die Drehmomente der Arme (2, 4) sich gerade kompensieren.

3. Röntgenuntersuchungsgerät nach Anspruch 1 oder 2, dadurch gekennzeichnet, daß die Arme über zwei Zahnräder (6) mit dem gleichen Durchmesser miteinander gekoppelt sind.

4. Röntgenuntersuchungsgerät nach einem der vorhergehenden Ansprüche, dadurch gekennzeichnet, daß der Röntgenstrahler (1) um eine zur Drehachse eines der Arme (2) senkrechte Achse drehbar mit diesem Arm verbunden ist.

## Claims

1. An X-ray examination apparatus provided with a C- or U-shaped support which carries at its ends an X-ray source (1) and an imaging device (5) which are centered relative to each other, and which is pivotable about an axis (7) which is perpendicular to the connecting line (12) between the source and the imaging device, characterized in that the support comprises two arms (2, 4), one arm carrying the X-ray source (1) and the other arm carrying the imaging device (5), both arms being arranged so as to be pivotable about axes (8, 9) which extend at least substantially parallel to the connecting line (12) and being coupled to one another in such a way that if one arm is pivoted, the other arm is pivoted with the same angular velocity but in the opposite direction.

2. An X-ray examination apparatus as claimed in Claim 1, <u>characterized in that</u> the distance between the pivotal axes (8, 9) of the arms and the connecting line (12) is such that in a horizontal position of the pivotal axes (8, 9) the torques of the arms (2, 4) exactly compensate for each other.

3. An X-ray examination apparatus as claimed in Claim 1 or 2, <u>characterized in that</u> the arms are coupled to each other <u>via</u> two gearwheels (6) of the same diameter.

4. An X-ray apparatus as claimed in any one of the preceding Claims, <u>characterized in that</u> the X-ray source (1) is connected to one of the arms (2) so as to be rotatable about an axis which is perpendicular to the pivotal axis of said arm.

**Revendications**

1. Appareil d'examen à rayons X comportant un support en forme de C ou de U, sur les extrémités duquel sont montés, centrés l'un par rapport à l'autre, un générateur de rayons X (1) et un dispositif de prise de vues (5), et qui peut pivoter autour d'un axe perpendiculaire à la ligne de jonction (12) reliant le générateur de rayons X et le dispositif de prise de vues (5), caractérisé en ce que le support comporte deux bras (2, 4) dont le premier porte le générateur de rayons X (1) et l'autre, le dispositif de prise de vues (5), que les deux bras sont montés de manière à pouvoir pivoter autour d'axes (8, 9) au moins approximativement parallèles à la ligne de jonction (12) et sont couplés l'un à l'autre d'une manière telle que, lors d'un pivotement du premier bras, l'autre pivote à la même vitesse angulaire, mais dans uns sens opposé.

2. Appareil d'examen à rayons X suivant la revendication 1, caractérisé en ce que la distance séparant les axes de pivotement (8, 9) des bras de la ligne de jonction (12) est telle que, dans une position horizontale des axes de pivotement (8, 9), les couples de rotation des bras (2, 4) se compensent exactement.

3. Appareil d'examen à rayons X suivant la revendication 1 ou 2, caractérisé en ce que les bras sont couplés l'un à l'autre par l'intermédiaire de deux roues dentées (6) de même diamètre.

4. Appareil d'examen à rayons X suivant l'une quelconque des revendications précédentes, caractérisé en ce que le générateur de rayons X (1) est relié à un bras (2) de manière à pouvoir tourner autour d'un axe perpendiculaire à l'axe de pivotement de ce bras.

FIG. 1

FIG. 2

FIG. 5

FIG. 3a

FIG. 3b

FIG. 4a

FIG. 4b